(19) Europäisches Patentamt
European Patent Office
Office européen des brevets

(11) Veröffentlichungsnummer: **0 384 250 A2**

(12) **EUROPÄISCHE PATENTANMELDUNG**

(21) Anmeldenummer: **90102642.7**

(51) Int. Cl.[5]: **C07D 417/12, C07D 417/14, C07D 413/12, A01N 43/72**

(22) Anmeldetag: **10.02.90**

(30) Priorität: **24.02.89 DE 3905763**

(43) Veröffentlichungstag der Anmeldung: **29.08.90 Patentblatt 90/35**

(84) Benannte Vertragsstaaten: **BE CH DE DK ES FR GB IT LI NL SE**

(71) Anmelder: **BAYER AG**

**D-5090 Leverkusen 1 Bayerwerk(DE)**

(72) Erfinder: **Wolf, Hilmar, Dr.**
**Haus Gravener Strasse 105**
**D-4018 Langenfeld(DE)**
Erfinder: **Wroblowsky, Heinz-Jürgen, Dr.**
**Galdbacher Strasse 34**
**D-4018 Langenfeld(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Lürssen, Klaus, Dr.**
**August-Kierspel-Strasse 145**
**D-5060 Bergisch Gladbach 2(DE)**

(54) **Sulfonylierte Azinyliminoheteroazole, Verfahren und Zwischenprodukte zu ihrer Herstellung und ihre Verwendung als Herbizide.**

(57) Die Erfindung betrifft neuartige sulfonylierte Azinyliminoheteroazole der allgemeinen Formel (I),

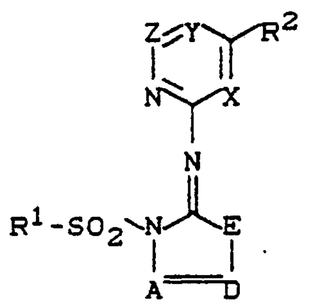

(I)

in welcher
A für Stickstoff oder die Gruppierung C-$A^1$ steht,
D für Stickstoff oder die Gruppierung C-$D^1$ steht,
(mit der Maßgabe, daß A und D nicht beide gleichzeitig für Stickstoff stehen),
E für Sauerstoff oder Schwefel steht,
X für Stickstoff oder eine CH-Gruppierung steht,
Y für Stickstoff oder die Gruppierung C-$R^3$ steht und
Z für Stickstoff oder die Gruppierung C-$R^4$ steht,
(wobei die Reste $A^1$, $D^1$, $R^1$, $R^2$, $R^3$ und $R^4$ die in der Beschreibung angegebenen Bedeutungen haben), ein Verfahren und neue Zwischenprodukte zu ihrer Herstellung und ihre Verwendung als Herbizide.

EP 0 384 250 A2

# Sulfonylierte Azinyliminoheteroazole, Verfahren und Zwischenprodukte zu ihrer Herstellung und ihre Verwendung als Herbizide

Die Erfindung betrifft neuartige sulfonylierte Azinyliminoheteroazole, ein Verfahren und neue Zwischenprodukte zu ihrer Herstellung sowie ihre Verwendung als Herbizide.

Es ist bekannt, daß 3-Amino-1,2,4-triazol (Amitrol) als Herbizid verwendet werden kann (vgl. Science 145 (1964),497). Die Wirkung dieser Verbindung ist jedoch nicht in allen Belangen zurfriedenstellend.

Es wurden nun die neuen sulfonylierten Azinyliminoheteroazole der allgemeinen Formel (I)

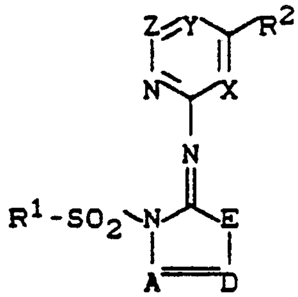

(I)

in welcher

A für Stickstoff oder die Gruppierung C-$A^1$ steht, worin

$A^1$ für Wasserstoff, Halogen, Cyano, Nitro oder für jeweils gegebenenfalls substituiertes Alkyl, Alkoxy, Alkylthio, Alkylsulfinyl, Alkylsulfonyl, Alkylamino, Dialkylamino oder Phenyl steht,

D für Stickstoff oder die Gruppierung C-$D^1$ steht, worin

$D^1$ für Wasserstoff, Halogen, Cyano, Nitro oder für jeweils gegebenenfalls substituiertes Alkyl, Alkoxy, Alkylthio, Alkylsulfinyl, Alkylsulfonyl, Alkylamino, Dialkylamino oder Phenyl steht,

(mit der Maßgabe, daß A und D nicht beide gleichzeitig für Sticktoff stehen),

E für Sauerstoff oder Schwefel steht,

$R^1$ für jeweils gegebenenfalls substituiertes Alkyl, Aralkyl, Aryl oder Heteroaryl steht,

$R^2$ für Wasserstoff, Halogen, Hydroxy, Amino oder für jeweils gegebenenfalls substituiertes Alkyl, Alkoxy, Alkylthio, Alkylsulfinyl, Alkylsulfonyl, Alkylamino oder Dialkylamino steht,

X für Stickstoff oder eine CH-Gruppierung steht,

Y für Stickstoff oder die Gruppierung C-$R^3$ steht, worin

$R^3$ für Wasserstoff, Halogen, Cyano, Alkyl, Formyl, Alkylcarbonyl oder Alkoxycarbonyl steht, und

Z für Stickstoff oder die Gruppierung C-$R^4$ steht, worin

$R^4$ für Wasserstoff, Halogen, Hydroxy, Amino oder für jeweils gegebenenfalls substituiertes Alkyl, Alkoxy, Alkylthio, Alkylsulfinyl, Alkylsulfonyl, Alkylamino oder Dialkylamino steht,

gefunden.

Man erhält die neuen sulfonylierten Azinyliminoheteroazole der allgemeinen Formel (I), wenn man Azinylaminoheteroazole der allgemeinen Formel (II)

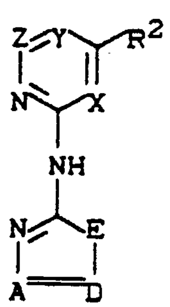

(II)

in welcher

A, D, E, $R^2$, X, Y und Z die oben angegebenen Bedeutungen haben,

mit Sulfonsäurehalogeniden oder Sulfonsäureanhydriden der allgemeinen Formel (III)

$R^1$-$SO_2$-Q (III)

in welcher

$R^1$ die oben angegebene Bedeutung hat und

Q für Fluor, Chlor, Brom oder die Gruppierung -O-$SO_2$-$R^1$ steht, worin

$R^1$ die oben angegebene Bedeutung hat,

gegebenenfalls in Gegenwart eines Säureakzeptors und gegebenenfalls in Gegenwart eines Verdünnungsmittels umsetzt.

Die neuen sulfonylierten Azinyliminoheteroazole der allgemeinen Formel (I) zeichnen sich durch starke herbizide Wirksamkeit aus. Diese Verbindungen stellen eine chemisch neuartige Klasse von Herbiziden dar.

Überraschenderweise zeigen die neuen Verbindungen der Formel (I) erheblich bessere herbizide Wirkung als das strukturell ähnliche bekannte Herbizid Aminotriazol (Amitrol).

Gegenstand der Erfindung sind vorzugsweise Verbindungen der Formel (I), in welcher

A für Stickstoff oder die Gruppierung C-$A^1$ steht, worin

$A^1$ für Wasserstoff, Fluor, Chlor, Brom, Iod, Cyano, Nitro oder für jeweils gegebenenfalls durch Fluor und/oder Chlor oder $C_1$-$C_4$-Alkoxy substituiertes $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkylamino oder Di-($C_1$-$C_4$)-alkyl-amino oder für gegebenenfalls durch Fluor, Chlor, Brom, $C_1$-$C_4$-Alkyl, $C_1$-$C_2$-Fluor-und/oder -Chlor-alkyl, $C_1$-$C_4$-Alkoxy und/oder $C_1$-$C_2$-Fluor- und/oder Chlor-alkoxy substituiertes Phenyl steht,

D für Stickstoff oder die Gruppierung C-$D^1$ steht, worin

$D^1$ für Wasserstoff, Fluor, Chlor, Brom, Iod, Cyano, Nitro oder für jeweils gegebenenfalls durch Fluor und/oder Chlor oder $C_1$-$C_4$-Alkoxy substituiertes $C_1$-$C_4$-Alkyl, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkylamino oder Di-($C_1$-$C_4$)-alkylamino oder für gegebenenfalls durch Fluor, Chlor, Brom, $C_1$-$C_4$-Alkyl, $C_1$-$C_2$-Fluor-und/oder Chlor-alkyl, $C_1$-$C_4$-Alkoxy und/oder $C_1$-$C_2$-Fluor- und/oder Chlor-alkoxy substituiertes Phenyl steht,

(mit der Maßgabe, daß A und D nicht beide gleichzeitig für Stickstoff stehen),

E für Sauerstoff oder Schwefel steht,

$R^1$ für den Rest

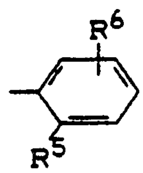

steht, worin

$R^5$ und $R^6$ gleich oder verschieden sind und für Wasserstoff, Fluor, Chlor, Brom, Iod, Cyano, Nitro, $C_1$-$C_6$-Alkyl [welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)aminocarbonyl, Hydroxy, $C_1$-$C_4$-Alkoxy, Formyl oxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist], für $C_2$-$C_6$-Alkenyl [welches gegebenenfalls durch Fluor, Chlor, Brom, Cyano, $C_1$-$C_4$-Alkoxy-carbonyl, Carboxy oder Phenyl substituiert ist], für $C_2$-$C_6$-Alkinyl [welches gegebenenfalls durch Fluor, Chlor, Brom, Cyano, $C_1$-$C_4$-Alkoxy-carbonyl, Carboxy oder Phenyl substituiert ist], für $C_1$-$C_4$-Alkoxy [welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist], für $C_1$-$C_4$-Alkylthio [welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist], für $C_3$-$C_6$-Alkenyloxy [welches gegebenenfalls durch Fluor, Chlor, Brom, Cyano oder $C_1$-$C_4$-Cyano oder $C_1$-$C_4$-Alkoxy-carbonyl substituiert ist], für $C_2$-$C_6$-Alkenylthio [welches gegebenenfalls durch Fluor, Chlor, Brom, Cyano, Nitro, $C_1$-$C_3$-Alkylthio oder $C_1$-$C_4$-Alkoxy- carbonyl substituiert ist], $C_3$-$C_6$-Alkinyloxy, $C_3$-$C_6$-Alkinylthio oder für den Rest-$S(O)_pR^7$ stehen, wobei

p für die Zahlen 1 oder 2 steht und

$R^7$ für $C_1$-$C_4$-Alkyl [welches gegebenenfalls durch Fluor, Chlor, Brom, Cyano oder $C_1$-$C_4$-Alkoxy-carbonyl substituiert ist], $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest -NHOR$^8$ steht, wobei

$R^8$ für $C_1$-$C_6$-Alkyl [welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbo-

nyl oder Di-($C_1$-$C_4$-alkyl)-amino-carbonyl substituiert ist], für $C_3$-$C_6$-Alkenyl [welches gegebenenfalls durch Fluor, Chlor oder Brom substituiert ist], $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl [wel ches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist], für Benzhydryl oder für Phenyl [welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist] steht,

$R^5$ und $R^6$ weiterhin für Phenyl oder Phenoxy, für $C_1$-$C_4$-Alkylcarbonylamino, $C_1$-$C_4$-Alkoxy-carbonylamino, $C_1$-$C_4$-Alkylamino-carbonylamino, Di-($C_1$-$C_4$-alkyl)-amino-carbonylamino, oder für den Rest -CO-$R^9$ stehen, wobei

$R^9$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$-Alkylthio, Amino, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht [welche gegebenenfalls durch Fluor und/oder Chlor substituiert sind],

$R^5$ und $R^6$ weiterhin für $C_1$-$C_4$-Alkylsulfonyloxy, Di-($C_1$-$C_4$-alkyl)-aminosulfonylamino oder für den Rest -CH=N-$R^{10}$ stehen, wobei

$R^{10}$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/ oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

worin weiter

$R^1$ für den Rest

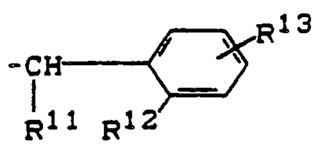

steht, worin

$R^{11}$ für Wasserstoff oder $C_1$-$C_4$-Alkyl steht,

$R^{12}$ und $R^{13}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Carboxy, $C_1$-$C_4$-Alkoxy-carbonyl, $C_1$-$C_4$-Alkylsulfonyl oder Di-($C_1$-$C_4$-alkyl)-aminosulfonyl stehen; worin weiter

$R^1$ für den Rest

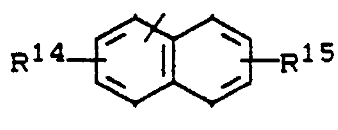

steht, worin

$R^{14}$ und $R^{15}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist] oder $C_1$-$C_4$-Alkoxy [welches gegebenenfalls durch Fluor und/oder Chlor substituiert ist], stehen; worin weiter

$R^1$ für den Rest

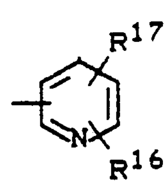

steht, worin

$R^{16}$ und $R^{17}$ gleich oder verschieden sind und für Wasserstoff, Fluor, Chlor, Brom, Nitro, Cyano, $C_1$-$C_4$-Alkyl [welches gegebenenfalls durch Fluor und/oder Chlor substituiert ist], $C_2$-$C_4$-Alkenyl [welches gegebenenfalls durch Fluor und/oder Chlor substituiert ist], $C_1$-$C_4$-Alkoxy [welches gegebenenfalls durch Fluor und/oder Chlor substituiert ist], für $C_1$ $C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], sowie für Di-($C_1$-$C_4$-alkyl)-aminosulfonyl, $C_1$-$C_4$-Alkoxy-carbonyl, Dimethylaminocarbonyl oder Dioxolanyl stehen; worin weiter

$R^1$ für den Rest

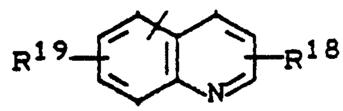

steht, worin

$R^{18}$ und $R^{19}$ gleich oder verschieden sind und für Wasserstoff, Fluor, Chlor, Brom, $C_1$-$C_4$-Alkyl [welches gegebenenfalls durch Fluor und/oder Brom substituiert ist], $C_1$-$C_4$-Alkoxy [welches gegebenenfalls durch Fluor und/oder Chlor substituiert ist], für $C_1$-$C_4$-Alkylthio, $C_1$-$C_4$-Alkylsulfinyl oder $C_1$-$C_4$-Alkylsulfonyl [welche gegebenenfalls durch Fluor und/oder Chlor substituiert sind], oder für Di-($C_1$-$C_4$-alkyl)-aminosulfonyl stehen; worin weiter

$R^1$ für den Rest

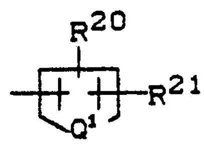

steht, worin

$R^{20}$ und $R^{21}$ gleich oder verschieden sind und für Wasserstoff, Fluor, Chlor, Brom, Cyano, Nitro, $C_1$-$C_4$-Alkyl [welches gegebenenfalls durch Fluor, Chlor, $C_1$-$C_4$-Alkoxy und/oder $C_1$-$C_4$-Halogenalkoxy substituiert ist], $C_1$-$C_4$-Alkoxy [welches gegebenenfalls durch Fluor und/ oder Chlor substituiert ist], $C_1$-$C_4$-Alkylthio, $C_1$-$C_4$-Alkylsulfinyl oder $C_1$-$C_4$-Alkylsulfonyl [welches gegebenenfalls durch Fluor und/oder Chlor substituiert ist], Di-($C_1$-$C_4$-alkyl)-amino-sulfonyl oder $C_1$-$C_4$-Alkoxy-carbonyl stehen, und

$Q^1$ für Sauerstoff, Schwefel oder die Gruppierung N-$Q^2$ steht, wobei

$Q^2$ für Wasserstoff, $C_1$-$C_4$-Alkyl [welches gegebenenfalls durch Fluor, Chlor, Brom oder Cyano substituiert ist], $C_3$-$C_6$-Cycloalkyl, Benzyl, Phenyl [welches gegebenenfalls durch Fluor, Chlor, Brom oder Nitro substituiert ist], $C_1$-$C_4$-Alkylcarbonyl, $C_1$-$C_4$-Alkoxy-carbonyl oder Di-($C_1$-$C_4$-alkyl)-aminocarbonyl steht; worin weiter

$R^1$ für den Rest

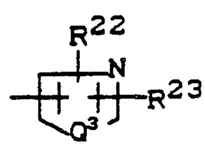

steht, worin

$R^{22}$ und $R^{23}$ gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,

$Q^3$ für Schwefel oder die Gruppierung N-$R^{24}$ steht, wobei

$R^{24}$ für Wasserstoff oder $C_1$-$C_4$-Alkyl steht; worin weiter

$R^1$ für den Rest

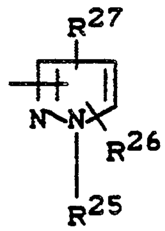

steht, worin
$R^{25}$ für Wasserstoff, $C_1$-$C_4$-Alkyl, Phenyl oder (Iso)Chinolinyl steht,
$R^{26}$ für Wasserstoff, Halogen, Cyano, Nitro, $C_1$-$C_4$-Alkyl [welches gegebenenfalls durch Fluor und/oder Chlor substituiert ist], $C_1$-$C_4$-Alkoxy [welches gegebenenfalls durch Fluor und/oder Chlor substituiert ist], Dioxolanyl oder $C_1$-$C_4$-Alkoxy-carbonyl steht und
$R^{27}$ für Wasserstoff, Halogen oder $C_1$-$C_4$-Alkyl steht; worin weiter
$R^1$ für den Rest

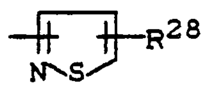

steht, worin
$R^{28}$ für Wasserstoff, Halogen, $C_1$-$C_4$-Alkyl oder $C_1$-$C_4$-Alkoxy-carbonyl steht; worin weiter
$R^1$ für den Rest

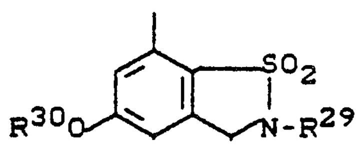

steht, worin
$R^{29}$ für $C_1$-$C_4$-Alkyl steht und
$R^{30}$ für $C_1$-$C_4$-Alkyl steht, worin weiter
$R^1$ für den Rest

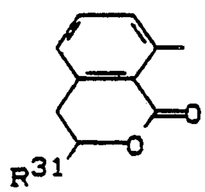

steht, worin
$R^{31}$ für Wasserstoff oder Methyl steht,
in welcher weiter
$R^2$ für Wasserstoff, Fluor, Chlor, Brom, Hydroxy, Amino, $C_1$-$C_4$-Alkyl, $C_1$-$C_4$-Halogenalkyl, $C_1$-$C_2$-Alkoxy-$C_1$-$C_2$-alkyl, Bis-($C_1$-$C_2$-alkoxy)-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Alkylsulfinyl, $C_1$-$C_4$-Alkylsulfonyl, $C_1$-$C_4$-Alkylamino, Dimethylamino oder Diethylamino steht,
X für Stickstoff oder eine CH-Gruppierung steht,
Y für Stickstoff oder die Gruppierung C-$R^3$ steht, worin
$R^3$ für Wasserstoff, Fluor, Chlor, Brom, Cyano, Methyl, Formyl, Acetyl, Methoxycarbonyl oder Ethoxycarbonyl steht, und
Z für Stickstoff oder die Gruppierung C-$R^4$ steht, worin
$R^4$ für Wasserstoff, Fluor, Chlor, Brom, Hydroxy, Amino, $C_1$-$C_4$-Alkyl, $C_1$-$C_4$-Halogenalkyl, $C_1$-$C_2$-Alkoxy-$C_1$-

$C_2$-alkyl, Bis-($C_1$-$C_2$-alkoxy)-$C_1$-$C_2$-Alkyl, $C_1$-$C_4$-Alkoxy, $C_1$-$C_4$-Halogenalkoxy, $C_1$-$C_4$-Alkylthio, $C_1$-$C_4$-Alkylsulfinyl, $C_1$-$C_4$-Alkylsulfonyl, $C_1$-$C_4$-Alkylamino, Dimethylamino oder Diethylamino steht.

Gegenstand der Erfindung sind insbesondere Verbindungen der Formel (1), in welcher

A für Stickstoff oder die Gruppierung C-$A^1$ steht, worin

$A^1$ für Wasserstoff, Methyl, Ethyl, Propyl, Isopropyl, Butyl, Isobutyl, sec-Butyl, tert-Butyl, Difluormethyl, Trifluormethyl, Dichlormethyl, Trichlormethyl, Chlordifluormethyl, Fluordichlormethyl, Methylthio, Ethylthio, Propylthio, Butylthio oder Phenyl steht,

D für Stickstoff oder die Gruppierung C-$D^1$ steht, worin

$D^1$ für Wasserstoff, Methyl, Ethyl, Propyl, Isopropyl, Butyl, Isobutyl, sec-Butyl, tert-Butyl, Difluormethyl, Trifluormethyl, Dichlormethyl, Trichlormethyl, Chlordifluormethyl, Fluordichlormethyl, Methylthio, Ethylthio, Propylthio, Butylthio oder Phenyl steht,

(mit der Maßgabe, daß A und D nicht beide gleichzeitig für Stickstoff stehen),

E für Sauerstoff oder Schwefel steht,

$R^1$ für den Rest

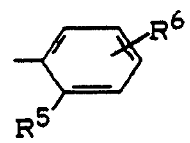

steht worin

$R^5$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, N-Methoxy-N-methylaminosulfonyl, Phenyl, Phenoxy oder $C_1$-$C_3$-Alkoxy-carbonyl steht und

$R^6$ für Wasserstoff, Fluor oder Chlor steht; worin weiter

$R^1$ für den Rest

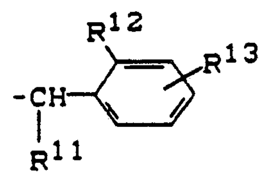

steht, worin

$R^{11}$ für Wasserstoff steht,

$R^{12}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und

$R^{13}$ für Wasserstoff, Fluor oder Chlor steht; worin weiter

$R^1$ für den Rest

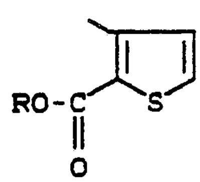

steht, worin

R für $C_1$-$C_4$-Alkyl steht, oder

$R^1$ für den Rest

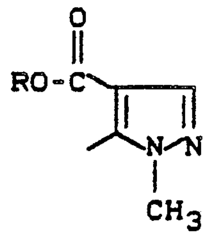

steht, worin
R für $C_1$-$C_4$-Alkyl steht, oder
$R^1$ für den Rest

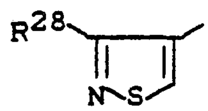

steht, worin
$R^{28}$ für Wasserstoff, Chlor, Methyl, Ethyl, Methoxycarbonyl oder Ethoxycarbonyl steht;
in welcher weiter
$R^2$ für Wasserstoff, Fluor, Chlor, Brom, Methyl, Ethyl, Trifluormethyl, Methoxy, Ethoxy, Difluormethoxy, Methylthio, Ethylthio, Methylamino, Ethylamino oder Dimethylamino steht,
X für Stickstoff oder eine CH-Gruppierung steht,
Y für Stickstoff oder die Gruppierung C-$R^3$ steht, worin
$R^3$ für Wasserstoff, Fluor, Chlor oder Methyl steht und
Z für Stickstoff oder die Gruppierung C-$R^4$ steht, worin
$R^4$ für Wasserstoff, Fluor, Chlor, Brom, Methyl, Ethyl, Trifluormethyl, Methoxy, Ethoxy, Propoxy, Isopropoxy, Difluormethoxy, Methylthio, Ethylthio, Methylamino, Ethylamino oder Dimethylamino steht.

Verwendet man beispielsweise 2-(4,6-Dimethoxy-pyrimidin-2-yl-amino)-oxazol und 2-Fluor-benzolsulfonsäurechlorid als Ausgangsstoffe, so kann der Reaktionsablauf beim erfindungsgemäßen Verfahren durch das folgende Formelschema skizziert werden:

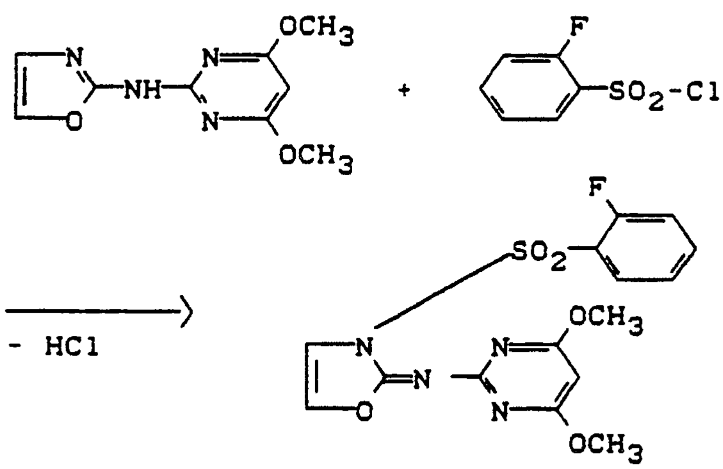


Die beim erfindungsgemäßen Verfahren zur Herstellung von Verbindungen der Formel (I) als Ausgangsstoffe zu verwendenden Azinylaminoheteroazole sind durch die Formel (II) allgemein definiert.

In Formel (II) haben A, D, E, $R^2$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, D, E, $R^2$, X, Y und Z angegeben wurden.

Beispiele für die Ausgangsstoffe der Formel (II) sind in der nachstehenden Tabelle 1 aufgeführt.

Tabelle 1: Beispiele für die Ausgangsstoffe der Formel (II)

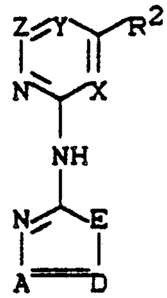

(II)

| A | D | E | $R^2$ | X | Y | Z |
|---|---|---|---|---|---|---|
| N | CH | O | $CH_3$ | N | CH | $C-CH_3$ |
| N | CH | S | $CH_3$ | N | CH | $C-CH_3$ |
| CH | N | O | $CH_3$ | N | CH | $C-CH_3$ |
| CH | N | S | $CH_3$ | N | CH | $C-CH_3$ |
| CH | CH | O | $CH_3$ | N | CH | $C-CH_3$ |
| CH | CH | S | $CH_3$ | N | CH | $C-CH_3$ |
| N | $C-CH_3$ | O | $CH_3$ | N | CH | $C-CH_3$ |
| N | $C-CH_3$ | S | $CH_3$ | N | CH | $C-CH_3$ |
| $C-CH_3$ | CH | S | $CH_3$ | N | CH | $C-CH_3$ |
| N | $C-CF_3$ | S | $CH_3$ | N | CH | $C-CH_3$ |
| N | $C-C_2H_5$ | S | $CH_3$ | N | CH | $C-CH_3$ |
| N | $C-C_6H_5$ | S | $CH_3$ | N | CH | $C-CH_3$ |

Tabelle 1 - Fortsetzung

| A | D | E | $R^2$ | X | Y | Z |
|---|---|---|---|---|---|---|
| N | $C-SCH_3$ | S | $CH_3$ | N | CH | $C-CH_3$ |
| $C-C_2H_5$ | CH | S | $CH_3$ | N | CH | $C-CH_3$ |
| N | $C-CHCl_2$ | S | $CH_3$ | N | CH | $C-CH_3$ |
| C-Cl | C-Cl | S | $CH_3$ | N | CH | $C-CH_3$ |
| N | CH | O | $CH_3$ | N | CH | $C-OCH_3$ |
| N | CH | S | $CH_3$ | N | CH | $C-OCH_3$ |
| CH | N | S | $CH_3$ | N | CH | $C-OCH_3$ |
| $C-CH_3$ | N | S | $CH_3$ | N | CH | $C-OCH_3$ |
| CH | CH | S | $CH_3$ | N | CH | $C-OCH_3$ |
| N | $C-CH_3$ | O | $CH_3$ | N | CH | $C-OCH_3$ |
| N | $C-CH_3$ | S | $CH_3$ | N | CH | $C-OCH_3$ |
| N | $C-SCH_3$ | S | $CH_3$ | N | CH | $C-OCH_3$ |
| N | CH | S | $OCH_3$ | N | CH | $C-OCH_3$ |
| CH | N | S | $OCH_3$ | N | CH | $C-OCH_3$ |
| $C-CH_3$ | N | S | $OCH_3$ | N | CH | $C-OCH_3$ |
| CH | CH | S | $OCH_3$ | N | CH | $C-OCH_3$ |
| N | $C-CH_3$ | O | $OCH_3$ | N | CH | $C-OCH_3$ |
| N | $C-CH_3$ | S | $OCH_3$ | N | CH | $C-OCH_3$ |
| N | $C-CF_3$ | S | $OCH_3$ | N | CH | $C-OCH_3$ |
| N | $C-SCH_3$ | S | $OCH_3$ | N | CH | $C-OCH_3$ |
| N | $C-C_6H_5$ | S | $OCH_3$ | N | CH | $C-OCH_3$ |

Tabelle 1 - Fortsetzung

| A | D | E | $R^2$ | X | Y | Z |
|---|---|---|---|---|---|---|
| N | $C-CH(CH_3)_2$ | S | $OCH_3$ | N | CH | $C-OCH_3$ |
| N | $C-SC_4H_9$ | S | $OCH_3$ | N | CH | $C-OCH_3$ |
| C-Cl | C-Cl | S | $OCH_3$ | N | CH | $C-OCH_3$ |
| N | CH | S | $OCH_3$ | N | CH | C-Cl |
| CH | N | S | $OCH_3$ | N | CH | C-Cl |
| $C-CH_3$ | N | S | $OCH_3$ | N | CH | C-Cl |
| CH | CH | S | $OCH_3$ | N | CH | C-Cl |
| N | $C-CH_3$ | S | $OCH_3$ | N | CH | C-Cl |
| N | $C-SCH_3$ | S | $OCH_3$ | N | CH | C-Cl |
| N | CH | S | H | N | CH | $C-CH_3$ |
| CH | N | S | H | N | CH | $C-CH_3$ |
| $C-CH_3$ | N | S | H | N | CH | $C-CH_3$ |
| CH | CH | S | H | N | CH | $C-CH_3$ |
| N | $C-CH_3$ | S | H | N | CH | $C-CH_3$ |
| N | CH | S | $CF_3$ | N | CH | $C-OCH_3$ |
| CH | N | S | $CF_3$ | N | CH | $C-OCH_3$ |
| $C-CH_3$ | N | S | $CF_3$ | N | CH | $C-OCH_3$ |
| N | $C-CH_3$ | S | $CF_3$ | N | CH | $C-OCH_3$ |
| CH | CH | S | $CF_3$ | N | CH | $C-OCH_3$ |
| N | $C-SCH_3$ | S | $CF_3$ | N | CH | $C-OCH_3$ |
| N | CH | S | $OCH_3$ | N | CH | $C-OCHF_2$ |

Tabelle 1 - Fortsetzung

| A | D | E | $R^2$ | X | Y | Z |
|---|---|---|---|---|---|---|
| CH | N | S | $OCH_3$ | N | CH | $C-OCHF_2$ |
| $C-CH_3$ | N | S | $OCH_3$ | N | CH | $C-OCHF_2$ |
| N | $C-CH_3$ | S | $OCH_3$ | N | CH | $C-OCHF_2$ |
| N | CH | S | $CH_3$ | N | CH | $C-OCHF_2$ |
| CH | N | S | $CH_3$ | N | CH | $C-OCHF_2$ |
| $C-CH_3$ | N | S | $CH_3$ | N | CH | $C-OCHF_2$ |
| N | $C-CH_3$ | S | $CH_3$ | N | CH | $C-OCHF_2$ |
| CH | CH | S | $CH_3$ | N | CH | $C-OCHF_2$ |
| N | $C-SCH_3$ | S | $CH_3$ | N | CH | $C-OCHF_2$ |
| N | $C-C_6H_5$ | S | $CH_3$ | N | CH | $C-OCHF_2$ |
| N | CH | S | $OCHF_2$ | N | CH | $C-OCHF_2$ |
| CH | N | S | $OCHF_2$ | N | CH | $C-OCHF_2$ |
| $C-CH_3$ | N | S | $OCHF_2$ | N | CH | $C-OCHF_2$ |
| N | $C-CH_3$ | S | $OCHF_2$ | N | CH | $C-OCHF_2$ |
| CH | CH | S | $OCHF_2$ | N | CH | $C-OCHF_2$ |
| N | $C-SCH_3$ | S | $OCHF_2$ | N | CH | $C-OCHF_2$ |
| N | $C-C_6H_5$ | S | $OCHF_2$ | N | CH | $C-OCHF_2$ |
| C-Cl | C-Cl | S | $OCHF_2$ | N | CH | $C-OCHF_2$ |
| N | CH | S | $CH_3$ | N | N | $C-CH_3$ |
| CH | N | S | $CH_3$ | N | N | $C-CH_3$ |
| $C-CH_3$ | N | S | $CH_3$ | N | N | $C-CH_3$ |

Tabelle 1 - Fortsetzung

| A | D | E | $R^2$ | X | Y | Z |
|---|---|---|---|---|---|---|
| N | $C-CH_3$ | S | $CH_3$ | N | N | $C-CH_3$ |
| CH | CH | S | $CH_3$ | N | N | $C-CH_3$ |
| C-Cl | C-Cl | S | $CH_3$ | N | N | $C-CH_3$ |
| N | CH | S | $CH_3$ | N | N | $C-OCH_3$ |
| CH | N | S | $CH_3$ | N | N | $C-OCH_3$ |
| $C-CH_3$ | N | S | $CH_3$ | N | N | $C-OCH_3$ |
| N | $C-CH_3$ | S | $CH_3$ | N | N | $C-OCH_3$ |
| CH | CH | S | $CH_3$ | N | N | $C-OCH_3$ |
| N | $C-SCH_3$ | S | $CH_3$ | N | N | $C-OCH_3$ |
| N | $C-CF_3$ | S | $CH_3$ | N | N | $C-OCH_3$ |
| N | $C-C_6H_5$ | S | $CH_3$ | N | N | $C-OCH_3$ |
| N | CH | S | $OCH_3$ | N | N | $C-OCH_3$ |
| CH | N | S | $OCH_3$ | N | N | $C-OCH_3$ |
| $C-CH_3$ | N | S | $OCH_3$ | N | N | $C-OCH_3$ |
| N | $C-CH_3$ | S | $OCH_3$ | N | N | $C-OCH_3$ |
| N | $C-SCH_3$ | S | $OCH_3$ | N | N | $C-OCH_3$ |
| N | $C-CF_3$ | S | $OCH_3$ | N | N | $C-OCH_3$ |
| N | $C-C_6H_5$ | S | $OCH_3$ | N | N | $C-OCH_3$ |
| CH | CH | S | $OCH_3$ | N | N | $C-OCH_3$ |
| C-Cl | C-Cl | S | $OCH_3$ | N | N | $C-OCH_3$ |
| N | CH | S | $OCH_3$ | N | N | C-Cl |

Tabelle 1 - Fortsetzung

| A | D | E | $R^2$ | X | Y | Z |
|---|---|---|---|---|---|---|
| N | CH | S | $OC_2H_5$ | N | N | $C-OC_2H_5$ |
| N | CH | S | $C_2H_5$ | N | N | $C-OCH_3$ |

Die Ausgangsstoffe der Formel (II) sind noch nicht aus der Literatur bekannt.

Man erhält die neuen Verbindungen der Formel (II) wenn man

α) Aminoheteroazole der allgemeinen Formel (IV)

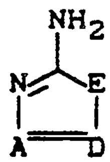
(IV)

in welcher

A, D und E die oben angegebenen Bedeutungen haben,

mit Azinen der allgemeinen Formel (V)

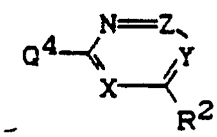
(V)

in welcher

$R^2$, X, Y und Z die oben angegebenen Bedeutungen haben und

$Q^4$ für Halogen oder Alkylsulfonyl steht,

gegebenenfalls in Gegenwart eines Säureakzeptors, wie z.B Kaliumcarbonat, und gegebenenfalls in Gegenwart eines Verdünnungsmittels, wie z.B. Acetonitril, Tetrahydrofuran, Dioxan oder Dimethylformamid, bei Temperaturen zwischen 0 °C und 150 °C umsetzt, oder wenn man

ß) Heteroazole der allgemeinen Formel (VI)

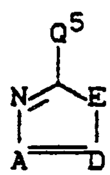
(VI)

in welcher

A, D und E die oben angegebenen Bedeutungen haben und

$Q^5$ für Halogen oder Alkylsulfonyl steht,

mit Aminoazinen der allgemeinen Formel (VII)

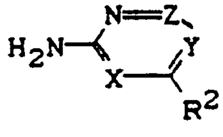
(VII)

in welcher

$R^2$, X, Y und Z die oben angegebenen Bedeutungen haben,
gegebenenfalls in Gegenwart eines Säureakzeptors, wie z.B. Kaliumcarbonat, und gegebenenfalls in Gegenwart eines Verdünnungsmittels, wie z.B. Acetonitril, Tetrahydrofuran, Dioxan oder Dimethylformamid, bei Temperaturen zwischen 0 °C und 150 °C umsetzt.

Die als Zwischenprodukte benötigten Aminoheteroazole sind durch die Formel (IV) allgemeinen definiert. In Formel (IV) haben A, D und E vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, D und E angegeben wurden.

Als Beispiele für die Verbindungen der Formel (IV) seien genannt:
2-Amino-oxazol, 2-Amino-thiazol, 2-Amino-1,3,4-thiadiazol, 5-Amino-1,2,4-thiadiazol, 2-Amino-1,3,4-oxadiazol, 2-Amino-4-methyl-thiazol, 2-Amino-5-methyl-thiazol, 2-Amino-5-methyl-1,3,4-thiadiazol, 2-Amino-5-ethyl-1,3,4-thiadiazol, 2-Amino-5-trifluormethyl-1,3,4-thiadiazol, 2-Amino-5-difluormethyl-1,3,4-thiadiazol, 2-Amino-5-dichlormethyl-1,3,4-thiadiazol, 2-Amino-5-methylthio-1,3,4-thiadiazol und 2 Amino-5-phenyl-1,3,4-thiadiazol.

Die Aminoheteroazole der Formel (IV) sind bekannt und/oder können nach an sich bekannten Verfahren hergestellt werden (vgl. Comprehensive Heterocyclic Chemistry, Vol. 6, Pergamon Press 1984).

Die weiter als Zwischenprodukte benötigten Azine sind durch die Formel (V) allgemein definiert. In Formel (V) haben $R^2$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^2$, X, Y und Z angegeben wurden, und $Q^4$ steht vorzugsweise für Fluor, Chlor, Brom oder $C_1$-$C_4$-Alkylsulfonyl, insbesondere für Chlor oder Methylsulfonyl.

Als Beispiele für die Verbindungen der Formel (V) seien genannt:
2-Chlor- und 2-Methylsulfonyl-4,6-dimethyl-pyrimidin, -4-methyl-6-methoxy-pyrimidin, -4,6-dimethoxy-pyrimidin, -4-methyl-6-ethoxy-pyrimidin, -4-chlor-6-methoxy-pyrimidin, -4-methyl-pyrimidin, -4-chlor-6-methyl-pyrimidin, -4-trifluormethyl-6-methoxy-pyrimidin, -4-methoxy-6-difluormethoxy-pyrimidin, -4-methyl-6-difluormethoxy-pyrimidin, -4,6-bis-difluormethoxy-pyrimidin, -4-chlor-6-ethoxy-pyrimidin, -4,6-diethoxy-pyrimidin, -4,5-dichlor-6-methyl-pyrimidin, -4-methyl-5-chlor-6-methoxy-pyrimidin, -4,6-dichlor-pyrimidin, -4-ethyl-6-methoxy-pyrimidin, -5-chlor-4,6-dimethoxy-pyrimidin sowie -4,6-bis-trifluormethyl-pyrimidin, ferner 2-Chlor-4,6-dimethyl-s-triazin 2-Chlor-4-methyl-6-methoxy-s-triazin, 2-Chlor-4,6-dimethoxy-s-triazin, 2,4-Dichlor-6-methoxy-s-triazin, 2-Chlor-4-ethyl-6-methoxy-s-triazin und 2-Chlor-4-methyl-6-ethoxy-s-triazin.

Die Azine der Formel (V) sind bekannt und/oder können nach an sich bekannten Verfahren hergestellt werden (vgl. J. Chem. Soc. 1957, 1830, 1833; J. Org. Chem 26 (1961), 792; US-P 3 308 119 und US-P 4 711 959).

Die weiter als Zwischenprodukte benötigen Heteroazole sind durch die Formel (VI) allgemein definiert. In Fromel (VI) haben A, D und E vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, D und E angegeben wurden und $Q^5$ steht vorzugsweise für Fluor, Chlor, Brom oder $C_1$-$C_4$-Alkylsulfonyl, insbesondere für Chlor oder Methylsulfonyl.

Als Beispiele für die Verbindungen der Formel (VI) seien genannt:
2-Chlor-oxazol, 2-Chlor-thiazol, 2-Chlor- und 2-Methylsulfonyl-1,3,4-thiadiazol, 2-Chlor-1,3,4-oxadiazol, 5-Chlor-1,2,4-thiadiazol, 2-Chlor-4-methyl-thiazol, 2,4,5-Trichlor-thiazol, 2-Chlor-5-methyl-thiazol, 2-Chlor- und 2-Methylsulfonyl-5-methyl-1,3,4-thiadiazol, 2-Chlor- und 2-Methylsulfonyl-5-ethyl-1,3,4-thiadiazol, 2-Chlor- und 2-Methylsulfonyl-5-trifluormethyl-1,3,4-thiadiazol, 2-Chlor- ud 2-Methylsulfonyl-5-difluormethyl-1,3,4-thiadiazol, 2-Chlor- und 2-Methylsulfonyl-5-dichlormethyl-1,3,4-thiadiazol, 2-Chlor- und 2-Methylsulfonyl-5-methylthio-1,3,4-thiadiazol und 2-Chlor-5-phenyl-1,3,4-thiadiazol.

Die Heteroazole der Formel (VI) sind bekannt und/oder können nach an sich bekannten Verfahren hergestellt werden (vgl. Comprehensive Heterocyclic Chemistry, Vol 6, Pergamon Press 1984).

Die weiter als Zwischenprodukte benötigten Aminoazine sind durch die Formel (VII) allgemein definiert. In Formel (VII) haben $R^2$, X, Y und Z vorzugsweise bzw. insbesondere diejenigen Bedeutungen, die bereits oben im Zusammenhang mit der Beschreibung der erfindungsge mäßen Verbindungen der Formel (I) vorzugsweise bzw. als insbesondere bevorzugt für $R^2$, X, Y und Z angegeben wurden.

Als Beispiele für die Verbindungen der Formel (VII) seien genannt:
2-Amino-4,6-dimethyl-pyrimidin, -4-methyl-6-methoxy-pyrimidin, -4,6-dimethoxy-pyrimidin, -4-methyl-6-ethoxy-pyrimidin, -4-ethyl-6-ethoxy-pyrimidin, -4-chlor-6-methoxy-pyrimidin, -4-methyl-pyrimidin, -4-chlor-6-methyl-pyrimidin, -4-trifluormethyl-6-methoxy-pyrimidin, -4-methyl-6-difluormethoxy-pyrimidin, -4-methoxy-6-difluormethoxy-pyrimidin, -4,6-bis-difluormethoxy-pyrimidin, -4-chlor-6-ethoxy-pyrimidin, -4,6-diethoxy-pyrimidin, -4,5-dichlor-6-methyl-pyrimidin, -4-methyl-5-chlor-6-methoxy-pyrimidin, -4,6-dichlor-pyrimidin, 5-chlor-4,6-bis-methoxy-pyrimidin und -4,6-bis-trifluormethyl-pyrimidin, ferner 2-Amino-4,6-dimethyl-s-triazin,

-4-methyl-6-methoxy-s-triazin, -4,6-dimethoxy-s-triazin, -4-chlor-6-methoxy-s-triazin, -4-ethyl-6-methoxy-s-triazin, -4-methyl-6-ethoxy-s-triazin, -4,6-diethoxy-s-triazin und -5,6-dimethyl-1,3,4-triazin.

Die Verbindungen der Formel (VII) sind bekannt und/oder können nach an sich bekannten Verfahren hergestellt werden (vgl. Chem. Pharm. Bull. 11 (1963), 1382; US-P 4 299 960).

Die beim erfindungsgemäßen Verfahren zur Herstellung der neuen Verbindungen der Formel (I) weiter als Ausgangs stoffe zu verwendenden Sulfonsäurehalogenide bzw. -anhydride sind durch die Formel (III) allgemein definiert. In Formel (III) hat $R^1$ vorzugsweise bzw. insbesondere diejenige Bedeutung, die oben im Rahmen der Beschreibung der erfindungsgemäßen Verbindungen der Formel (I) vorzugsweise bzw. als insbesondere bevorzugt angegeben wurde und Q steht vorzugsweise für Chlor.

Als Ausgangsstoffe der Formel (III) seien beispielsweise genannt:
Benzolsulfonsäurechlorid, 2-Chlor-, 3-Chlor-, 4-Chlor-, 2,5-Dichlor-, 2-Fluor-, 4-Fluor-, 2-Brom-, 4-Brom-, 2-Cyano-, 2-Nitro-, 4-Nitro-, 2-Methyl-, 4-Methyl-, 2-Chlormethyl-, 2-Trifluormethyl-, 2-Methoxy-, 4-Methoxy-, 2-Methylthio-, 2-Trifluormethylthio-, 2-Difluormethylthio-, 2-Cyclopropyloxycarbonyl-, 2-Phenoxy-, 2-Difluormethoxy-, 2-Trifluormethoxy-, 2-(2-Chlorethoxy)-, 2-Methylthiomethyl-, 2-Dimethylaminosulfonyl-, 2-Phenyl-, 2-Methoxycarbonyl-, 2-Ethoxycarbonyl-, 2-Dimethylaminocarbonyl- und 2-Diethylaminocarbonyl-benzolsulfonsäurechlorid sowie (2-Chlor-phenyl)-, (2-Cyano-phenyl)-, (2-Methoxycarbonyl-phenyl)- und (2-Trifluormethoxy-phenyl)-methansulfonsäurechlorid, 2-Chlor-6-methyl-benzolsulfonsäurechlorid und 2,6-Dichlor-benzolsulfonsäurechlorid.

Die Sulfonsäurehalogenide bzw. -anhydride der Formel (III) sind bekannt und/oder können nach an sich bekannten Verfahren hergestellt werden (vgl. J. Org. Chem. 33 (1968), 2104; J. Org. Chem. 25 (1960), 1824; DE-AS 2 308 262; EP-OS 23 140, 23 141, 23 422, 35 893, 48 143, 51 466, 64 322, 70 041, 44 808 und 44 809; US-PS 2 929 820, 4 282 242; 4 348 220 und 4 372 778 sowie Angew. Chem 93 (1981), 151).

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und-ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid und Pyridin.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetallhydroxide wie z B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert.-butylat, ferner aliphatische, aromatische oder heterocyclische Amine, beispielsweise Triethylamin, Trimethylamin, Dimethylanilin, Dimethylbenzylamin, Pyridin, Picolin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen -50 °C und +100 °C, vorzugsweise bei Temperaturen zwischen -20 °C und +40 °C.

Zur Durchführung des erfindungsgemäßen Verfahrens setzt man je Mol Azinylaminoheteroazol der Formel (II) im allgemeinen zwischen 1 und 5 Mol, vorzugsweise zwischen 1 und 4 Mol, Sulfonsäurehalogenid bzw. Sulfonsäureanhydrid der Formel (III) ein.

Die Reaktionskomponenten können in beliebiger Reihenfolge zusammengegeben werden.
In einer bevorzugten Ausführungsform des erfindungsgemäßen Verfahrens werden die Ausgangsstoffe der Formeln (II) und (III) bei Raumtemperatur mit einem Verdünnungsmittel verrührt, und in diese Mischung wird -gegebenenfalls nach Abkühlen - der Säureakzeptor langsam eindosiert Das Reaktionsgemisch wird dann bis zum Ende der Umsetzung gerührt.

Die Aufarbeitung kann auf übliche Weise durchgeführt werden, beispielsweise indem man - gegebenenfalls nach Einengen und/oder Verdünnen mit einem mit Wasser praktisch nicht mischbaren organischen Lösungsmittel, wie z B. Methylenchlorid - mit Wasser wäscht, trocknet, filtriert und vom Filtrat das Lösungsmittel sorgfältig abdestilliert. Das im Rückstand verbleibende Rohprodukt kann auf übliche Weise, z. B. durch Säulenchromatographie und/oder durch Umkristallisation, weiter gereinigt werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen

Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur Bekämpfung von monokotylen und dikotylen Unkräutern in monokotylen Kulturen sowohl im Vorauflauf- als auch im Nachauflauf-Verfahren.

Einige der erfindungsgemäßen Verbindungen zeigen auch fungizide Wirkung, insbesondere gegen Pyricularia oryzae an Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlo rierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von

Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methyl thio-1,2,4-triazin-5(4H)-on (METRIBUZIN) zur Unkrautbekämpfung in Sojabohnen, in Frage. Ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 2-Chlor-4-ethylamino-6-isopropylamino-1,3,5-triazin (ATRAZIN); 3,5-Dibrom-4-hydroxy-benzonitril (BROMOXYNIL); 4-Amino-6-t-butyl-3-ethylthio-1,2,4-triazin-5(4H)-on (ETHIOZIN); N-Phosphonomethylglycin (GLYPHOSATE); 3-Cyclohexyl-6-dimethylamino-1-methyl-1,3,5-triazin-2,4-dion (HEKAZINONE); 2-(4,5-Dihydro-4-methyl-4-isopropyl-5-oxo-1H-imidazol-2-yl)-pyridin-3-carbonsäure (IMAZAPYR); 3,5-Diiod-4-hydroxybenzonitril (IOXYNIL); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 2-Chlor-4,6-bis-(ethylamino)-1,3,5-triazin (SIMAZIN); Methyl-2-{[(4,6-dimethyl-2-pyrimidinyl)-aminocarbonyl]-aminosulfonyl}-benzoat (SULFOMETURON); 2,6-Dinitro-4-trifluormethyl-N,N-dipropylanilin (TRIFLURALIN); Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

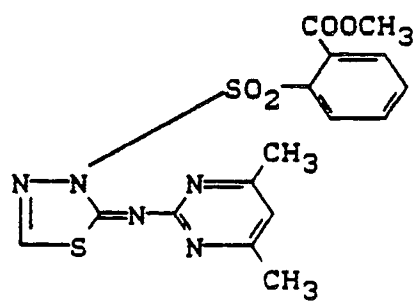

Zu einer Suspension von 2,1 g (0,01 Mol) 2-(4,6-Dimethyl-pyrimidin-2-yl-amino)-1,3,4-thiadiazol in 100 ml Dichlormethan werden 7,0 g (0,03 Mol) 2-Methoxycarbonylbenzolsulfonsäurechlorid gegeben und in diese Mischung werden dann unter Rühren und Kühlen mit Eiswasser 3 g (0,05 Mol) Trimethylamin eingeleitet. Die Reaktionsmischung wird 20 Stunden bei 20 °C bis 25 °C gerührt, anschließend mit gesättigter Natriumbicarbonatlösung gewaschen, über Magnesiumsulfat getrocknet und filtriert. Vom Filtrat wird das Lösungsmittel im Wasserstrahlvakuum abdestilliert und der Rückstand mit Methanol verrührt Das kristalline Produkt wird durch Absaugen isoliert

Man erhält 3,0 g (75 % der Theorie) 2-(4,6-Dimethylpyrimidin-2-yl-imino)-3-(2-methoxycarbonyl-phenylsulfonyl)-2,3-dihydro-1,3,4-thiadiazol vom Schmelzpunkt (unter Zersetzung) 160 °C.

Analog Beispiel 1 und entsprechend der allgemeinen Beschreibung des erfindungsgemäßen Verfahrens können die in der nachstehenden Tabelle 2 aufgeführten Verbindungen der Formel (I) hergestellt werden.

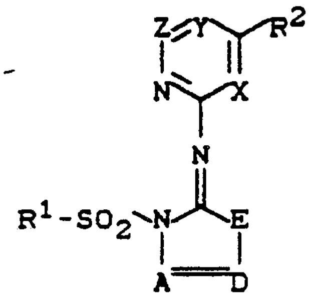

(I)

Tabelle 2:

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 2 | CH | CH | S | 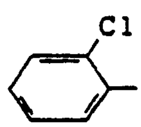 | $CH_3$ | N | CH | $C-CH_3$ | 188 |
| 3 | N | CH | S | 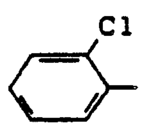 | $CH_3$ | N | CH | $C-CH_3$ | 254 |

Tabelle 2 - Fortsetzung

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 4 | CH | CH | S | 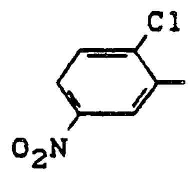 | $CH_3$ | N | CH | $C-CH_3$ | 129 |
| 5 | CH | CH | S | 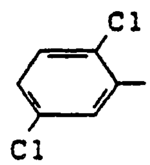 | $CH_3$ | N | CH | $C-CH_3$ | |
| 6 | CH | CH | S | 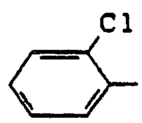 | $OCH_3$ | N | CH | $C-OCH_3$ | 176 |
| 7 | CH | CH | S | 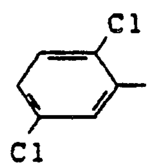 | $OCH_3$ | N | CH | $C-OCH_3$ | 250 |
| 8 | CH | CH | S | 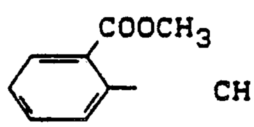 | $CH_3$ | N | CH | $C-CH_3$ | 174 |
| 9 | CH | CH | S | 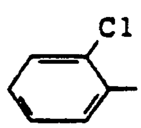 | $CH_3$ | N | CH | $C-OCH_3$ | 193 |
| 10 | CH | CH | S | 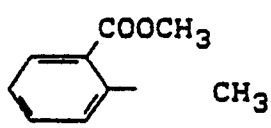 | $CH_3$ | N | CH | $C-OCH_3$ | 191 |

Tabelle 2 - Fortsetzung

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 11 | CH | CH | S | 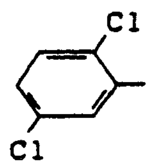 | $CH_3$ | N | CH | C-$OCH_3$ | 161 |
| 12 | CH | CH | S | 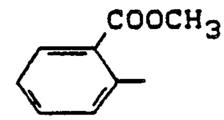 | $OCH_3$ | N | CH | C-$OCH_3$ | 147 |
| 13 | N | CH | S | 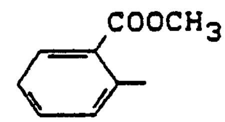 | $CH_3$ | N | CH | C-$OCH_3$ | 168 |
| 14 | CH | CH | S | 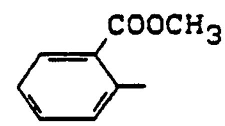 | $OCH_3$ | N | N | C-$OCH_3$ | 168 |
| 15 | CH | CH | S | 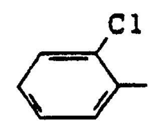 | $OCH_3$ | N | N | C-$OCH_3$ | 178 |
| 16 | N | CH | S | 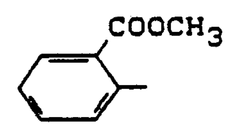 | $OCH_3$ | N | CH | C-$OCH_3$ | 156 |
| 17 | N | CH | S | 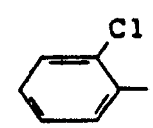 | $OCH_3$ | N | CH | C-$OCH_3$ | 169 |

Tabelle 2 - Fortsetzung

Beispiele für die Verbindungen der Formel (I)

| Bsp. Nr. | A | D | E | $R^1$ | $R^2$ | X | Y | Z | Schmelz-punkt °C |
|---|---|---|---|---|---|---|---|---|---|
| 18 | N | CH | S | 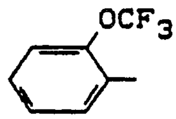 | $CH_3$ | N | CH | $C-CH_3$ | 178 |
| 19 | CH | CH | S | 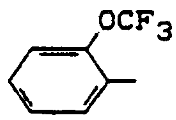 | $CH_3$ | N | CH | $C-CH_3$ | 178 |
| 20 | CH | CH | S | 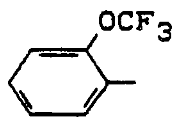 | $CH_3$ | N | CH | $C-OCH_3$ | 171 |
| 21 | N | CH | S | 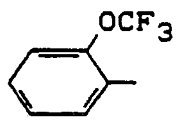 | $CH_3$ | N | CH | $C-OCH_3$ | 174 |
| 22 | N | CH | S | 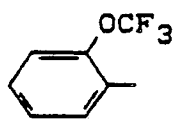 | $OCH_3$ | N | CH | $C-OCH_3$ | 128 |
| 23 | CH | CH | S | 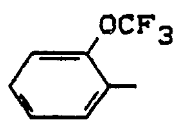 | $OCH_3$ | N | CH | $C-OCH_3$ | 155 |
| 24 | CH | CH | S | 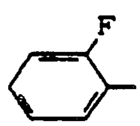 | $OCH_3$ | N | CH | $C-OCH_3$ | 173 |

Tabelle 2 - Fortsetzung

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 25 | N | CH | S | 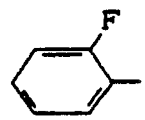 | $CH_3$ | N | CH | $C-CH_3$ | 158 |
| 26 | CH | CH | S | 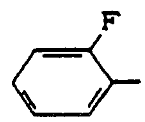 | $CH_3$ | N | CH | $C-CH_3$ | 197 |
| 27 | N | CH | S | 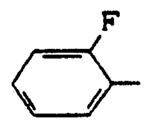 | $OCH_3$ | N | CH | $C-OCH_3$ | 168 |
| 28 | N | CH | S | 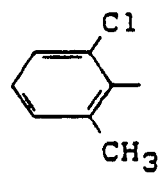 | $CH_3$ | N | CH | $C-CH_3$ | 168 |
| 29 | N | CH | S | 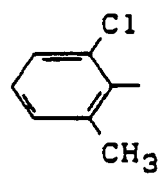 | $CH_3$ | N | CH | $C-OCH_3$ | 177 |
| 30 | N | $C-CH_3$ | S | 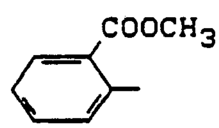 | $CH_3$ | N | CH | $C-CH_3$ | 171 |
| 31 | N | $C-CH_3$ | S | 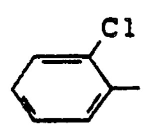 | $CH_3$ | N | CH | $C-CH_3$ | 188 |

Tabelle 2 - Fortsetzung

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 32 | N | $C-CH_3$ | S | 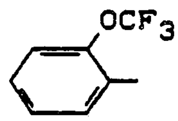 | $CH_3$ | N | CH | $C-CH_3$ | 179 |
| 33 | CH | CH | S | 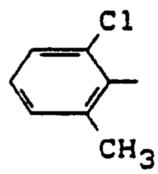 | $CH_3$ | N | CH | $C-OCH_3$ | 190 |
| 34 | CH | CH | S | 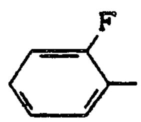 | $CH_3$ | N | CH | $C-OCH_3$ | 160 |
| 35 | N | $C-CH_3$ | S | 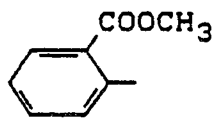 | $OCH_3$ | N | CH | $C-OCH_3$ | 170 |
| 36 | N | $C-CH_3$ | S | 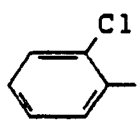 | $CH_3$ | N | CH | $C-OCH_3$ | 150 |
| 37 | N | $C-CH_3$ | S | 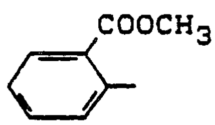 | $CH_3$ | N | CH | $C-OCH_3$ | 173 |
| 38 | N | $C-CH_3$ | S | 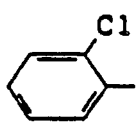 | $CH_3$ | N | CH | $C-OCH_3$ | 198 |

Tabelle 2 - Fortsetzung

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 39 | N | CH | S | 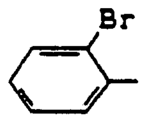 | $CH_3$ | N | CH | $C-OCH_3$ | 180 |
| 40 | N | CH | S | 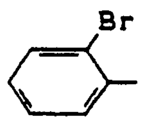 | $OCH_3$ | N | CH | $C-OCH_3$ | 140 |
| 41 | N | $C-C_2H_5$ | S | 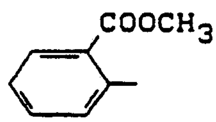 | $CH_3$ | N | CH | $C-OCH_3$ | 161 |
| 42 | N | $C-C_2H_5$ | S | 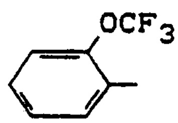 | $CH_3$ | N | CH | $C-OCH_3$ | 150 |
| 43 | N | CH | S | 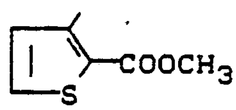 | $CH_3$ | N | CH | $C-CH_3$ | 158 |
| 44 | N | CH | S | 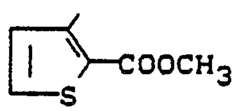 | $CH_3$ | N | CH | $C-OCH_3$ | 168 |
| 45 | N | CH | S | 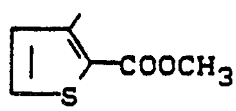 | $OCH_3$ | N | CH | $C-OCH_3$ | 148 |
| 46 | N | CH | S | 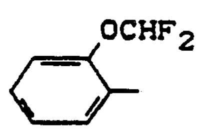 | $CH_3$ | N | CH | $C-OCH_3$ | 153 |

Tabelle 2 - Fortsetzung

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 47 | N | CH | S | 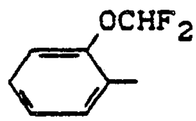 | $CH_3$ | N | CH | $C-OCH_3$ | 167 |
| 48 | N | CH | S | 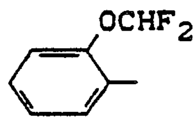 | $OCH_3$ | N | CH | $C-OCH_3$ | 160 |
| 49 | N | $C-SCH_3$ | S | 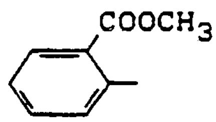 | $CH_3$ | N | CH | $C-CH_3$ | 160 |
| 50 | N | CH | S | 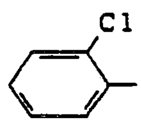 | $CH_3$ | N | CH | $C-OCH_3$ | 175 |
| 51 | N | CH | S | 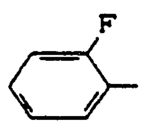 | $CH_3$ | N | CH | $C-OCH_3$ | 170 |
| 52 | N | CH | S | 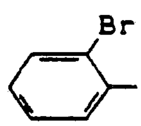 | $CH_3$ | N | CH | $C-CH_3$ | 200 |
| 53 | N | CH | S | 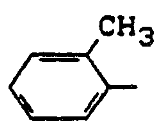 | $CH_3$ | N | CH | $C-OCH_3$ | |

Tabelle 2 - Fortsetzung

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 54 | N | CH | S | 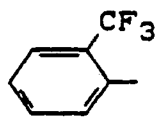 | $CH_3$ | N | CH | $C-CH_3$ | |
| 55 | N | CH | S | 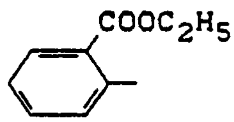 | $CH_3$ | N | CH | $C-CH_3$ | |
| 56 | N | CH | S | 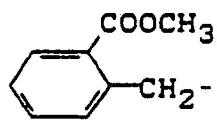 | $OCH_3$ | N | CH | $C-OCH_3$ | |
| 57 | N | CH | S | 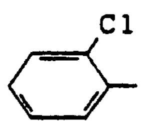 | $CH_3$ | N | N | $C-OCH_3$ | |
| 58 | N | CH | S | 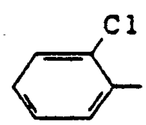 | $OCH_3$ | N | N | $C-OCH_3$ | |
| 59 | N | CH | S | 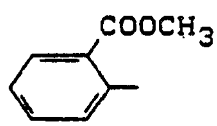 | $CH_3$ | N | N | $C-OCH_3$ | |
| 60 | N | CH | S | 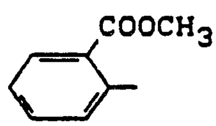 | $OCH_3$ | N | N | $C-OCH_3$ | |

Tabelle 2 - Fortsetzung

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 61 | N | CH | S | 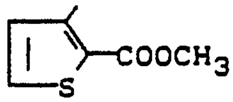 | $CH_3$ | N | N | C-$OCH_3$ | |
| 62 | N | CH | S | 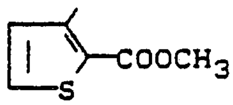 | $OCH_3$ | N | N | C-$OCH_3$ | |
| 63 | N | CH | S | 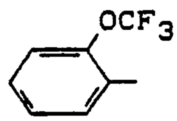 | $OCH_3$ | N | N | C-$OCH_3$ | |
| 64 | CH | CH | S | 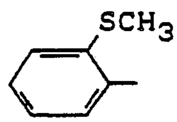 | $OCH_3$ | N | CH | C-$OCH_3$ | |
| 65 | N | CH | S | 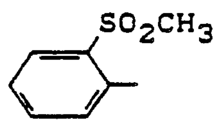 | $OCH_3$ | N | N | C-$OCH_3$ | |
| 66 | N | CH | S | 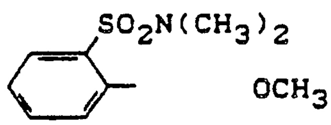 | $OCH_3$ | N | CH | C-$OCH_3$ | |
| 67 | N | CH | S | 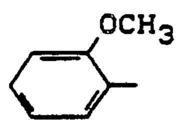 | $CH_3$ | N | CH | C-$CH_3$ | |
| 68 | N | CH | S | 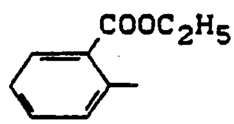 | $OCH_3$ | N | CH | C-Cl | |

Tabelle 2 - Fortsetzung

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 69 | N | CH | S | 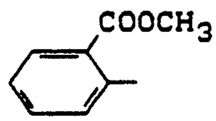 | $OCHF_2$ | N | CH | $C\text{-}OCHF_2$ | |
| 70 | N | CH | S | 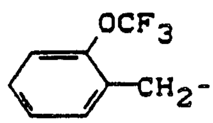 | $OCH_3$ | N | CH | $C\text{-}OCH_3$ | |
| 71 | N | CH | S | 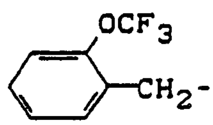 | $OCH_3$ | N | N | $C\text{-}OCH_3$ | |
| 72 | N | CH | S | 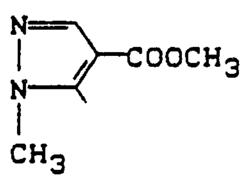 | $OCH_3$ | N | CH | $C\text{-}OCH_3$ | |
| 73 | N | CH | S | 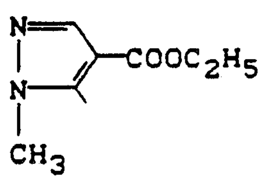 | $OCH_3$ | N | CH | $C\text{-}OCH_3$ | |
| 74 | N | CH | S | 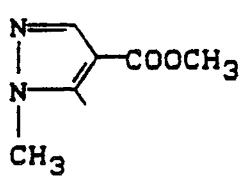 | $OCH_3$ | N | N | $C\text{-}OCH_3$ | |

Tabelle 2 - Fortsetzung

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 75 | N | CH | S | 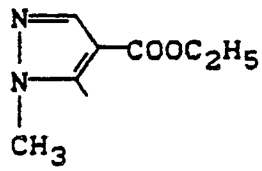 | $OCH_3$ | N | N | $C-OCH_3$ | |
| 76 | N | CH | S | 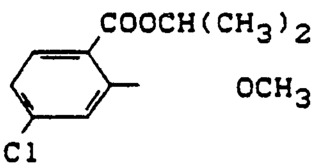 | $OCH_3$ | N | N | $C-OCH_3$ | |
| 77 | N | CH | S | 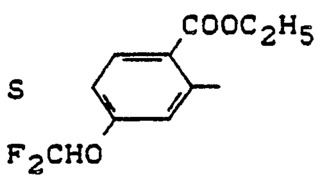 | $OCH_3$ | N | CH | $C-OCH_3$ | |
| 78 | N | CH | S | 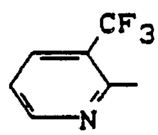 | $OCH_3$ | N | CH | $C-OCH_3$ | |
| 79 | N | CH | S | 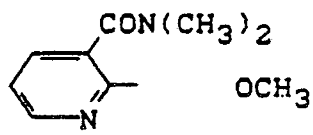 | $OCH_3$ | N | CH | $C-OCH_3$ | |
| 80 | N | CH | S | 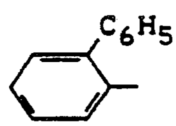 | $OCH_3$ | N | CH | $C-OCH_3$ | |

Tabelle 2 - Fortsetzung

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 81 | N | $C-CH_3$ | O | 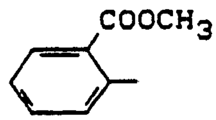 | $CH_3$ | N | CH | $C-OCH_3$ | |
| 82 | N | $C-CH_3$ | O | 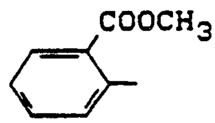 | $OCH_3$ | N | CH | $C-OCH_3$ | |
| 83 | C-Cl | N | O | 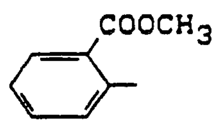 | $OCH_3$ | N | CH | $C-OCH_3$ | 97 |
| 84 | C-Cl | N | O | 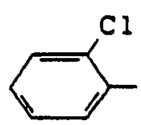 | $OCH_3$ | N | CH | $C-OCH_3$ | 125 |
| 85 | C-Cl | N | O | 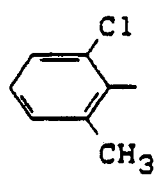 | $OCH_3$ | N | CH | $C-OCH_3$ | 152 |
| 86 | $C-CH_3$ | N | S | 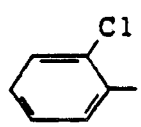 | $CH_3$ | N | CH | $C-OCH_3$ | 177 |
| 87 | $C-CH_3$ | N | S | 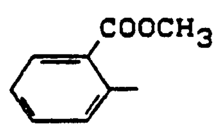 | $CH_3$ | N | CH | $C-OCH_3$ | 176 |

<u>Tabelle 2 - Fortsetzung</u>

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 88 | N | $C-CH_3$ | O | 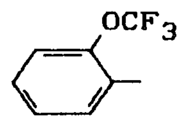 | $OCH_3$ | N | CH | $C-OCH_3$ | |
| 89 | N | CH | S | 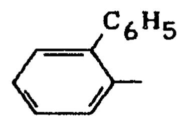 | $OCH_3$ | N | CH | $C-OCH_3$ | 170 |
| 90 | N | CH | S | 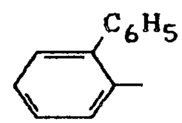 | $CH_3$ | N | CH | $C-OCH_3$ | 164 |
| 91 | N | $C-CH_3$ | O | 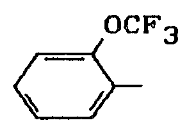 | $CH_3$ | N | CH | $C-OCH_3$ | |
| 92 | N | CH | S | 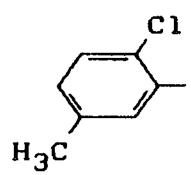 | $CH_3$ | N | CH | $C-OCH_3$ | |

<u>Tabelle 2 - Fortsetzung</u>

Beispiele für die Verbindungen der Formel (I)

| Bsp. Nr. | A | D | E | $R^1$ | $R^2$ | X | Y | Z | Schmelzpunkt °C |
|---|---|---|---|---|---|---|---|---|---|
| 93 | N | CH | S | 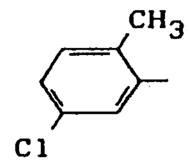 | $CH_3$ | N | CH | $C-OCH_3$ | |
| 94 | N | CH | S | 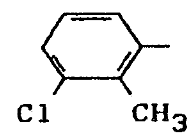 | $CH_3$ | N | CH | $C-CH_3$ | |
| 95 | N | CH | S | 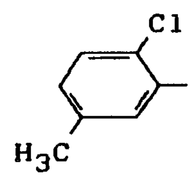 | $CH_3$ | N | CH | $C-CH_3$ | |
| 96 | N | CH | S | 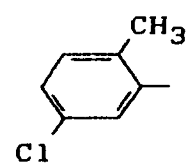 | $CH_3$ | N | CH | $C-CH_3$ | |
| 97 | N | CH | S | 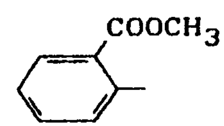 | $OCH_3$ | N | N | $C-OCH_3$ | |

<u>Ausgangsstoffe der Formel (II)</u>

Beispiel (II-1)

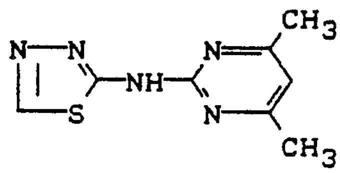

Eine Mischung aus 18,6 g (0,10 Mol) 2-Methylsulfonyl-4,6-dimethyl-pyrimidin, 14,0 g (0,10 Mol) Kaliumcarbonat, 10,1 g (0,10 Mol) 2-Amino-1,3,4-thiadiazol und 200 ml Acetonitril wird 30 Stunden unter Rückfluß zum Sieden erhitzt. Nach Abkühlen wird abgesaugt, der Feststoff mit 150 ml Wasser gewaschen und im Wasserstrahlvakuum bei 40 °C getrocknet.

Man erhält 19 g (92 i. der Theorie) 2-(4,6-Dimethylpyrimidin-2-yl-amino)-1,3,4-thiadiazol vom Schmelzpunkt >250 °C.

Analog können die in der nachstehenden Tabelle 3 aufgeführten Verbindungen der Formel (II) hergestellt werden.

Tabelle 3:

| Beispiele für die Verbingungen der Formel (II) | | | | | | | | |
|---|---|---|---|---|---|---|---|---|
| Bsp. Nr. | A | D | E | $R^2$ | X | Y | Z | Schmelzpunkt °C |
| II-2 | CH | CH | S | $CH_3$ | N | CH | C-$CH_3$ | 189 |
| II-3 | CH | CH | S | $OCH_3$ | N | N | C-$OCH_3$ | >250 |
| II-4 | CH | CH | S | $OCH_3$ | N | CH | C-$OCH_3$ | 213 |
| II-5 | N | CH | S | $OCH_3$ | N | CH | C-$OCH_3$ | 248 |
| II-6 | CH | CH | S | $CH_3$ | N | CH | C-$OCH_3$ | 197 |
| II-7 | N | CH | S | $CH_3$ | N | CH | C-$OCH_3$ | 231 |
| II-8 | C-$CH_3$ | CH | S | $CH_3$ | N | CH | C-$OCH_3$ | 165 |
| II-9 | N | C-$CF_3$ | S | $CH_3$ | N | CH | C-$CH_3$ | 250 |
| II-10 | N | C-$C_2H_5$ | S | $CH_3$ | N | CH | C-$OCH_3$ | 184 |
| II-11 | C-$CH_3$ | CH | S | $CH_3$ | N | CH | C-$CH_3$ | 134 |
| II-12 | N | C-$CH_3$ | S | $CH_3$ | N | CH | C-$CH_3$ | 223-225 |
| II-13 | N | C-$CH_3$ | S | $CH_3$ | N | CH | C-$OCH_3$ | 230 |
| II-14 | N | C-$CH_3$ | S | $OCH_3$ | N | CH | C-$OCH_3$ | 214 |
| II-15 | N | C-$C_6H_5$ | S | $CH_3$ | N | CH | C-$CH_3$ | 255 |
| II-16 | N | C-$SCH_3$ | S | $CH_3$ | N | CH | C-$CH_3$ | 194 |
| II-17 | N | C-$CH_3$ | O | $CH_3$ | N | CH | C-$OCH_3$ | |
| II-18 | N | C-$CH_3$ | O | $OCH_3$ | N | CH | C-$OCH_3$ | |

Anwendungsbeispiele

Beispiel A

Pre-emergence-Test

Lösungsmittel: 5 Gewichtsteile Aceton
Emulgator: 1 Gewichtsteil Alkylarylpolyglykolether
Zur Herstellung einer zweckmäßigen Wirkstoffzubereitung vermischt man 1 Gewichtsteil Wirkstoff mit

der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflänzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

Eine deutliche Überlegenheit in der Wirksamkeit gegenüber dem Stand der Technik zeigen in diesem Test z.B. die Verbindungen gemäß den Herstellungsbeispielen 1, 3 und 13.

**Ansprüche**

1. Sulfonylierte Azinyliminoheteroazole der allgemeinen Formel (I)

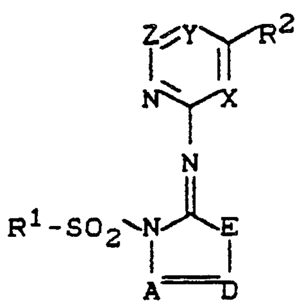

(I)

in welcher

A für Stickstoff oder die Gruppierung C-$A^1$ steht, worin

$A^1$ für Wasserstoff, Halogen, Cyano, Nitro oder für jeweils gegebenenfalls substituiertes Alkyl, Alkoxy, Alkylthio, Alkylsulfinyl, Alkylsulfonyl, Alkylamino, Dialkylamino oder Phenyl steht,

D für Stickstoff oder die Gruppierung C-$D^1$ steht, worin

$D^1$ für Wasserstoff, Halogen, Cyano, Nitro oder für jeweils gegebenenfalls substituiertes Alkyl, Alkoxy, Alkylthio, Alkylsulfinyl, Alkylsulfonyl, Alkylamino, Dialkylamino oder Phenyl steht,

(mit der Maßgabe, daß A und D nicht beide gleichzeitig für Sticktoff stehen),

E für Sauerstoff oder Schwefel steht,

$R^1$ für jeweils gegebenenfalls substituiertes Alkyl, Aralkyl, Aryl oder Heteroaryl steht,

$R^2$ für Wasserstoff, Halogen, Hydroxy, Amino oder für jeweils gegebenenfalls substituiertes Alkyl, Alkoxy, Alkylthio, Alkylsulfinyl, Alkylsulfonyl, Alkylamino oder Dialkylamino steht,

X für Stickstoff oder eine CH-Gruppierung steht,

Y für Stickstoff oder die Gruppierung C-$R^3$ steht, worin

$R^3$ für Wasserstoff, Halogen, Cyano, Alkyl, Formyl, Alkylcarbonyl oder Alkoxycarbonyl steht, und

Z für Stickstoff oder die Gruppierung C-$R^4$ steht, worin

$R^4$ für Wasserstoff, Halogen, Hydroxy, Amino oder für jeweils gegebenenfalls substi tuiertes Alkyl, Alkoxy, Alkylthio, Alkylsulfinyl, Alkylsulfonyl, Alkylamino oder Dialkylamino steht.

2. Verfahren zur Herstellung von sulfonylierten Azinyliminoheteroazolen der allgemeinen Formel (I) gemäß Anspruch 1, dadurch gekennzeichnet, daß man Azinylaminoheteroazole der allgemeinen Formel (II)

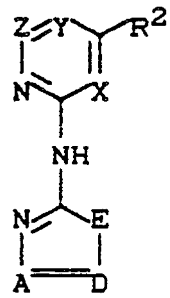

(II)

in welcher
A, D, E, $R^2$, X, Y und Z die in Anspruch 1 angegebenen Bedeutungen haben,
mit Sulfonsäurehalogeniden oder Sulfonsäureanhydriden der allgemeinen Formel (III)
$R^1$-$SO_2$-Q (III)
in welcher
$R^1$ die in Anspruch 1 angegebene Bedeutung hat und Q für Fluor, Chlor, Brom oder die Gruppierung -O-$SO_2$-$R^1$ steht, worin
$R^1$ die oben angegebene Bedeutung hat,
gegebenenfalls in Gegenwart eines Säureakzeptors und gegebenenfalls in Gegenwart eines Verdünnungsmittels umsetzt.

3. Herbizide Mittel, gekennzeichnet durch einen Gehalt an mindestens einem sulfonylierten Azinyliminoheteroazol der Formel (I) gemäß Anspruch 1.

4. Verfahren zur Bekämpfung von Unkräutern, dadurch gekennzeichnet, daß man sulfonylierte Azinyliminoheteroazole der Formel (I) gemäß Anspruch 1 auf die Unkräuter und/oder ihren Lebensraum einwirken läßt.

5. Verwendung von sulfonylierten Azinyliminoheteroazolen der Formel (I) gemäß Anspruch 1 zur Bekämpfung von Unkräutern.

6. Verfahren zur Herstellung von herbiziden Mitteln, dadurch gekennzeichnet, daß man sulfonylierte Azinyliminoheteroazole der Formel (I) gemäß Anspruch 1 mit Streckmitteln und/oder oberflächenaktiven Mitteln vermischt.

7. Azinylamino-heteroazole der allgemeinen Formel (II)

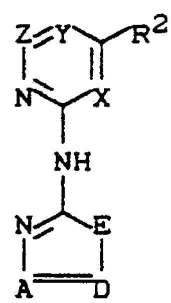

(II)

in welcher
A für Stickstoff oder die Gruppierung C-$A^1$ steht, worin
$A^1$ für Wasserstoff, Halogen, Cyano, Nitro oder für jeweils gegebenenfalls substituiertes Alkyl, Alkoxy, Alkylthio, Alkylsulfinyl, Alkylsulfonyl, Alkylamino, Dialkylamino oder Phenyl steht,
D für Stickstoff oder die Gruppierung C-$D^1$ steht, worin
$D^1$ für Wasserstoff, Halogen, Cyano, Nitro oder für jeweils gegebenenfalls substituiertes Alkyl, Alkoxy, Alkylthio, Alkylsulfinyl, Alkylsulfonyl, Alkylamino, Dialkylamino oder Phenyl steht,
(mit der Maßgabe, daß A und D nicht beide gleichzeitig für Sticktoff stehen),
E für Sauerstoff oder Schwefel steht,
$R^2$ für Wasserstoff, Halogen, Hydroxy, Amino oder für jeweils gegebenenfalls substituiertes Alkyl, Alkoxy, Alkylthio, Alkylsulfinyl, Alkylsulfonyl, Alkylamino oder Dialkylamino steht,
X für Stickstoff oder eine CH-Gruppierung steht,
Y für Stickstoff oder die Gruppierung C-$R^3$ steht, worin

$R^3$ für Wasserstoff, Halogen, Cyano, Alkyl, Formyl, Alkylcarbonyl oder Alkoxycarbonyl steht, und
Z für Stickstoff oder die Gruppierung C-$R^4$ steht, worin
$R^4$ für Wasserstoff, Halogen, Hydroxy, Amino oder für jeweils gegebenenfalls substituiertes Alkyl, Alkoxy, Alkylthio, Alkylsulfinyl, Alkylsulfonyl, Alkylamino oder Dialkylamino steht.